# EUROPÄISCHE PATENTSCHRIFT

(11) **EP 4 378 488 B1**
(45) Veröffentlichungstag und Bekanntmachung des Hinweises auf die Patenterteilung: **02.07.2025**
(21) Anmeldenummer: 22210431.7
(22) Anmeldetag: 30.11.2022
(51) Int. Cl.: A61L 9/22, A61B 5/055, A61B 6/03, A61B 6/00

(54) **BILDGEBUNGSVORRICHTUNG, LUFTKANALEINRICHTUNG UND VERFAHREN ZUM BETREIBEN EINER BILDGEBUNGSVORRICHTUNG**
IMAGING DEVICE, AIR DUCT DEVICE AND METHOD FOR OPERATING AN IMAGING DEVICE
DISPOSITIF D'IMAGERIE, DISPOSITIF DE CONDUIT D'AIR ET PROCÉDÉ DE FONCTIONNEMENT D'UN DISPOSITIF D'IMAGERIE

(43) Veröffentlichungstag der Anmeldung: 05.06.2024
(73) Patentinhaber: Siemens Healthineers AG, 91301 Forchheim (DE)
(72) Erfinder: Feher, Lambert, 76297 Stutensee (DE); Höcht, Philipp, 91207 Lauf (DE); Maciejewski, Bernd, 91477 Markt Bibart (DE); Meise, Florian, 91353 Hausen (DE); Seifert, Martin, 95447 Bayreuth (DE)
(74) Vertreter: Siemens Healthineers Patent Attorneys

(56) Entgegenhaltungen:
- CN-B- 105 497 930
- CN-U- 203 524 686
- CN-U- 215 607 847
- CN-U- 217 929 073
- DE-A1- 102020 216 423
- DE-U1- 202021 104 249

## Beschreibung

*Unabhängig vom grammatikalischen Geschlecht eines bestimmten Begriffes sind Personen mit männlicher, weiblicher oder anderer Geschlechteridentität mit umfasst.*

Die Erfindung betrifft eine Bildgebungsvorrichtung, eine Luftkanaleinrichtung sowie ein Verfahren zum Betreiben einer Bildgebungsvorrichtung.

Die Infektionsprävention in klinischen Umgebungen basiert auf zwei Hauptpfeilern, der Händehygiene (Desinfektion) und der Aufbereitung relevanter Oberflächen (Oberflächenhygiene). Durch die Reinigung und Desinfektion von Händen und relevanten Flächen werden potenzielle oder sichtbare Kontaminationen entfernt und das Infektionsrisiko verringert. Die Tröpfcheninfektion spielt eine entscheidende Rolle bei der Übertragung von Infektionskrankheiten, die auf Viren beruhen (z. B. Grippe, Windpocken oder Masern). Dabei werden die Erreger, die sich im Rachen oder in den Atemwegen befinden, durch winzige Speicheltröpfchen beim Niesen, Husten oder Sprechen in die Luft abgegeben. Diese Aerosole werden von anderen Menschen eingeatmet oder direkt über die Schleimhäute der oberen Atemwege aufgenommen.

Generell bestehen Aerosole aus festen oder flüssigen Partikeln unterschiedlicher Größe. Ein großer Teil sind solche im 100-um-Bereich. Es kann aber auch von einem Anteil deutlich kleinerer Partikel, kleiner als 5 um oder kleiner als 2,5 µm ausgegangen werden Quelle: DGUV Regel 102-001, Ausgabe September 2019, zu "Regeln für Sicherheit und Gesundheit bei Tätigkeiten mit Biostoffen im Unterricht" der Deutschen Gesetzlichen Unfallversicherung e.V. DGUV. Während Tröpfchen von 100 µm Durchmesser etwa 6 Sekunden benötigen, um aus einer Höhe von 2 m auf den Boden zu sinken, benötigen Tröpfchen von 10 µm Durchmesser 10 Minuten für die gleiche Entfernung, Tröpfchen von 1 µm benötigen 16,6 Stunden Quelle: Kappstein, Ines. Nosokomiale Infektionen: Prävention, Labordiagnostik, antimikrobielle Therapie; 122 Tabellen. Deutschland, Thieme, 2009. Kleinere Partikel bleiben daher länger in der Luft, können sich über Luftbewegung im Raum verteilen und das Infektionsgeschehen beeinflussen.

Kleinere Partikel bleiben also länger in der Luft und können sich durch die Luftbewegung im Raum verteilen.

Die COVID-19-Pandemie hat das Thema Tröpfcheninfektion erneut in den Fokus der Öffentlichkeit gerückt und zum einen gezeigt, dass nicht nur immungeschwächte Menschen einem hohen Risiko für solche Infektionskrankheiten ausgesetzt sind, insbesondere wenn die Erkrankung unerkannt oder im Vorfeld unbekannt ist. Andererseits hat die Pandemie auch gezeigt, wie wirksam sinnvolle Präventionsmaßnahmen die Übertragung reduzieren können.

Bei einigen bildgebenden Verfahren besteht in einem begrenzten Untersuchungsraum z. B. bei der Aufnahmeröhre eines Magnetresonanztomographen ein erhöhtes Risiko einer Tröpfcheninfektion, die während der Untersuchung eines Patienten auftreten kann. Die folgenden Probleme können während dieser Untersuchung auftreten:
1. Kleinste Tröpfchen können sich ansammeln und durch natürliche Konvektion in der Aufnahmeröhre schweben.
2. Schwebende kleinste Tröpfchenaerosole können durch konvektive Luftströme oder thermische natürliche Konvektion aus der Aufnahmeröhre in den Untersuchungsraum transportiert werden, in welchem sich die Bildgebungsvorrichtung befindet.
3. Größere Aerosoltröpfchen können die Oberfläche in der Aufnahmeröhre und das Patientenhandhabungssystem kontaminieren

Bisher wurden geeignete, der jeweiligen Infektionskrankheit angepasste Hygiene- und Schutzmaßnahmen z.B. medizinischer Mund-Nasen-Schutz oder FFP2-Schutzfunktion, Ganzkörperanzug, etc. angewandt. Darüber hinaus gibt es auf dem Markt eine Reihe von Filterlösungen, z.B. auf Basis von HEPA-Filtern (High-Efficiency Particulate Air/Arrestance), UVC- oder plasmabasierten Verfahren, die die Raumluft entsprechend dekontaminieren. Auch Kombinationen der Geräte untereinander, im Folgenden als "Filteranlagen" bezeichnet, sind zu finden. Voraussetzung für diese Systeme ist jedoch, dass die Systemleistung sowie die Luftvolumenströme genau auf die jeweilige Anwendung abgestimmt sind. Auch eine Bestimmung der Belastung der kontaminierten Luft mittels Aerosolen trägt wesentlich zur gezielten Dekontamination der Luft bei.

Um Luft zu desinfizieren, sterilisieren oder zu reinigen, muss sie technisch in einem Luftstrom behandelt werden. Dieser Luftstrom hängt entscheidend von der Geometrie ab. Bei mechanischen Filtern muss die Luft unter hohem Druck und Energieaufwand bei hoher Geräuschentwicklung 4- bis 16-mal durch den Filter gepresst werden.

Wird die durchströmende Luft durch UV-Lampen behandelt, müssen die Anlagen mit einem moderaten Luftstrom ausgelegt werden. Gängige Ozonisatoren / Ionisatoren / Plasmasysteme benötigen umfangreiche Wechselwirkungskammern, was den Luftstrom ebenfalls begrenzt. Allen diesen Verfahren ist gemeinsam, dass sie aufgrund ihrer langgestreckten würfelförmigen oder länglichen Bauweise als zentrale Einheiten eingesetzt werden.

In jüngster Zeit werden auch mobile Oberflächen-Dekontaminationsgeräte oder stationäre UVC-Lampen zur Oberflächendekontamination eingesetzt. Ein Nachteil dieser auf dem Markt erhältlichen Lösungen ist die Implementierung in das MRT-System, die Größe der Einheit oder der benötigte Platz im Gebäude, um die Wirksamkeit des Dekontaminationsprozesses zu verbessern. Die marktgängigen Lösungen von basieren auf elektromagnetischen Entladungen und einer vergleichsweise langsamen Desinfektionswirkung durch Radikale. Zusätzlich können diese eine UV-Lampe umfassen.

In Bezug auf medizinische Geräte ist aus der CN203524686U eine CT-Maschine mit Selbstreinigungsfunktion bekannt. Hierbei ist eine Sterilisationseinheit auf Basis von UVC, Plasma oder Ozon für die CT-Maschine offenbart. Eine selektive Messung, Absaugung, Dekontaminierung und Ableitung potenziell kontaminierter Luft ist in der Veröffentlichung nicht offenbart.

Hier ist zu beachten, dass eine validierte Desinfektionsmaßnahme für eine Fläche zu einer Keimreduktion von 5 log-Stufen (EU) bzw. 6 log-Stufen (USA) führen muss. Auch die Wirkung auf behüllte oder unbehüllte Viren muss im Rahmen der Validierung mit geeigneten Methoden nachgewiesen werden. Bei UVCbasierten Verfahren ist die für eine effektive und effiziente Dekontamination der Luft erforderliche Dosisleistung stark von der Wellenlänge der verwendeten UVC-Quelle und der Verweilzeit der Raumluft in der entsprechenden Filtereinheit abhängig. Erfahrungen aus der Oberflächendekontamination mit UVC haben gezeigt, dass teilweise lange Bestrahlungszeiten in Abhängigkeit von der Entfernung und der Dosisleistung notwendig sind, um eine hohe Keimabtötung zu gewährleisten. Auch die Desinfektion ist bei plasmabasierten Geräten schwierig zu realisieren. Daher wird in den vorangegangenen und folgenden Ausführungen der Begriff "Dekontamination" verwendet.

DE 10 2020 216 423 A1 offenbart ein System zur Desinfektion von Oberflächen und/oder Raumluft. Hierzu ist zumindest eine UV-Quelle an einem medizinischen Gerät und/oder in einem medizinischen Untersuchungs- und/oder Behandlungsraum derart angeordnet, dass sie zur desinfizierenden Bestrahlung zumindest einer Oberfläche und/oder zumindest eines Luftstroms geeignet ist.

Aus DE 20 2021 104 249 A1 ist eine Magnetresonanzvorrichtung mit einer Luftzufuhreinheit bekannt. Die Luftzufuhreinheit ist zu einer Frischluftzufuhr in einen von einer Scannereinheit umgebenen Patientenaufnahmebereich der Magnetresonanzvorrichtung ausgebildet. Die Luftzufuhreinheit weist hierzu eine Luftdesinfektionseinheit auf.

Aus CN 203 524 686 U ist ein CT-Gerät bekannt mit einer Selbstreinigungsfunktion, die einen Rahmen und eine auf dem Rahmen integrierte Desinfektionsvorrichtung zum Empfangen von Desinfektions- und Kontrollinformationen umfasst und die entsprechende Desinfektions- und Kontrollfunktionen ausführt.

CN 215 607 847 A offenbart eine interne Luftdesinfektionsvorrichtung für ein medizinisches CT-Rack.

Es ist eine Aufgabe der Erfindung, eine Filtereinrichtung bereitzustellen, welche eine effizientere Dekontamination von Luft in einer Aufnahmeröhre einer Bildgebungsvorrichtung ermöglicht.

Diese Aufgabe wird gelöst durch den jeweiligen Gegenstand der unabhängigen Ansprüche. Vorteilhafte Weiterbildungen und bevorzugte Ausführungsformen sind Gegenstand der abhängigen Ansprüche.

Ein erster Aspekt der Erfindung betrifft eine Bildgebungsvorrichtung, aufweisend eine Aufnahmeröhre zur Aufnahme eines durch die Bildgebungsvorrichtung zu untersuchenden Objektes, wobei die Aufnahmeröhre eine, einer Aufnahmeöffnung der Aufnahmeröhre zugewandte erste Aufnahmenröhrenhälfte und eine der Aufnahmeöffnung abgewandte zweite Aufnahmenröhrenhälfte aufweist. Bei der Bildgebungsvorrichtung kann es sich beispielsweise um eine Bildgebungsvorrichtung zur Durchführung einer Computertomographie oder zur Durchführung einer Magnetresonanztomographie handeln. Die Aufnahmeöffnung kann als Tunnel ausgebildet sein, welcher durch die Bildgebungsvorrichtung zumindest teilweise umschlossen sein kann. Die Aufnahmeröhre kann dazu vorgesehen sein, einen Patienten oder ein zu untersuchendes Objekt aufzunehmen. Die Aufnahmeröhre kann die Aufnahmeöffnung aufweisen, durch welche der zu untersuchende Patient oder das zu untersuchende Objekt in die Aufnahmeröhre verfahren werden kann.

Das Untersuchungsobjekt kann sich während der Untersuchung durch die Bildgebungsvorrichtung in der Aufnahmeröhre befinden und dadurch in einem Erfassungsbereich der Bildgebungsvorrichtung angeordnet sein.

Es ist vorgesehen, dass die Bildgebungsvorrichtung eine Luftkanaleinrichtung aufweist, die dazu eingerichtet ist, Luft aus der Aufnahmeröhre an einer Ansaugeinrichtung der Luftkanaleinrichtung anzusaugen, die angesogene Luft zur Dekontamination durch eine Luftfiltereinrichtung der Luftkanaleinrichtung zu leiten und die dekontaminierte Luft an einer Ausgabeeinrichtung der Luftkanaleinrichtung in die Aufnahmeröhre auszugeben. Mit anderen Worten umfasst die Bildgebungsvorrichtung die Luftkanaleinrichtung, welche dazu eingerichtet ist, einen Luftaustausch in der Aufnahmeröhre zu bewirken. Zur Bewirkung des Luftaustausches weist die Luftkanaleinrichtung die Ansaugeinrichtung auf, welche dazu eingerichtet ist, die Luft aus der Aufnahmeröhre der Luftkanaleinrichtung zuzuführen. Die Ansaugeinrichtung kann beispielsweise Öffnungen oder Ansaugröhren umfassen, welche an einer Wand der Aufnahmeröhre angeordnet sein können. Die Ansaugeinrichtung kann ein Element zum Erzeugen eines Unterdrucks, beispielsweise einen Ventilator aufweisen, wodurch Luft der Aufnahmeröhre in die Luftkanaleinrichtung geführt werden kann. Die Luftkanaleinrichtung weist die Luftfiltereinrichtung auf, welche dazu vorgesehen ist, die angesogene Luft zu dekontaminieren. Die Luftkanaleinrichtung ist dazu eingerichtet, die aus der Aufnahmeröhre angesogene Luft durch die Luftfiltereinrichtung zu leiten, um die Dekontamination der Luft durch die Luftfiltereinrichtung zu ermöglichen. Das Dekontaminieren kann ein Inaktivieren von Keimen und/oder ein Herausfiltern von Aerosolen aus der angesogenen Luft durch die Luftfiltereinrichtung umfassen. Die Luftfiltereinrichtung kann als mechanische, elektrostatische, vorzugsweise plasmabasierte und/oder UVbasierte Filtereinrichtung eingerichtet sein. Die Luftkanaleinrichtung weist eine Ausgabeeinrichtung auf, welche dazu eingerichtet ist, die kontaminierte Luft aus der Luftkanaleinrichtung in die Aufnahmeröhre abzugeben. Die Ausgabeeinrichtung kann beispielsweise Öffnungen und oder Düsen an der Wand der Aufnahmeröhre umfassen, an welchen die Luft nach der Dekontamination aus der Luftkanaleinrichtung in die Aufnahmeröhre geführt wird.

Die Luftkanaleinrichtung ist dazu eingerichtet, einen Hauptluftstrom der Luft von der Ausgabeeinrichtung zu der Ansaugeinrichtung entlang der Aufnahmeröhre zu führen, wobei der Hauptluftstrom durch die Aufnahmeröhre in eine Richtung einer Aufnahmeöffnung der Aufnahmeröhre verläuft. Mit anderen Worten ist die Luftkanaleinrichtung dazu eingerichtet, einen Hauptluftstrom der Luft innerhalb der Aufnahmeröhre bereitzustellen. Der Hauptluftstrom der Luft verläuft innerhalb der Aufnahmeröhre von der Ausgabeeinrichtung zu der Ansaugeinrichtung. Der Hauptluftstrom wird durch die Luftkanaleinrichtung entlang einer Richtung geführt, welche zu der Aufnahmeöffnung der Aufnahmeröhre ausgerichtet ist. Es kann beispielsweise vorgesehen sein, dass die Ansaugeinrichtung bezüglich einer Längsrichtung der Aufnahmeröhre zwischen der Ausgabeeinrichtung und der Aufnahmeöffnung der Aufnahmeröhre angeordnet ist. Dadurch wird die von der Ausgabeeinrichtung ausgegebene Luft durch die Ansaugeinrichtung angesogen, wodurch der Hauptluftstrom, ausgehend von der Ausgabeeinrichtung in Richtung der Aufnahmeöffnung erzeugt wird. Der Hauptluftstrom verläuft in die Ansaugeinrichtung, sodass der Hauptluftstrom nicht durch die Aufnahmeöffnung aus der Aufnahmeröhre herausgeführt wird.

Durch die Erfindung ergibt sich der Vorteil, dass durch die Luftkanaleinrichtung in der Aufnahmeröhre der Hauptluftstrom erzeugt wird, welcher einen in der Aufnahmeröhre angeordneten Patienten mit dekontaminierter Luft versorgen kann. Gleichzeitig wird durch die Ansaugeinrichtung verhindert, dass kontaminierte Luft des Hauptstroms aus der Aufnahmeröhre entweicht und somit einen Raum, in welchem sich die Bildgebungsvorrichtung befinden kann, kontaminiert.

Die Erfindung umfasst auch Weiterbildungen, durch die sich weitere Vorteile ergeben.

Eine Weiterbildung der Erfindung sieht vor, dass die Luftkanaleinrichtung eine Luftbarriereneinrichtung aufweist, die dazu eingerichtet ist, zumindest einen Teil der dekontaminierten Luft zur Erzeugung einer Luftbarriere in der Aufnahmeröhre zwischen der Ansaugeinrichtung und der Aufnahmeöffnung auszugeben. Mit anderen Worten ist es vorgesehen, dass die Luftkanaleinrichtung dazu eingerichtet ist, die durch die Filtereinrichtung dekontaminierte Luft nicht alleine durch die Ausgabeeinrichtung in die Aufnahmeröhre zur Bereitstellung des Hauptluftstroms ausgegeben, sondern einen Teil der dekontaminierten Luft durch die Luftbarriereneinrichtung in die Aufnahmeröhre auszugeben. Die Luftbarriereneinrichtung ist dazu eingerichtet, mittels der Ausgabe der Luft die Luftbarriere in der Aufnahmeröhre bereitzustellen. Die Luftbarriereneinrichtung kann dazu eingerichtet sein, die durch die Luftbarriereneinrichtung auszugebende dekontaminierte Luft entlang einer oder mehrerer vorgegebener Richtungen mit vorgegebenen Geschwindigkeiten in die Aufnahmeröhre auszugeben, die derart parameterisiert sind, dass durch die ausgegebene Luft die Luftbarriere gebildet wird. Die Luft kann eine Barrierenströmung aufweisen, durch welche die Wirkung der Luftbarriere aufweisen kann. Die Barriereströmung kann ausgehend von der Luftbarriereneinrichtung zu der Ansaugeinrichtung innerhalb der Aufnahmeröhre verlaufen. Die Luftbarriereneinrichtung kann beispielsweise auf einer, der Ansaugeinrichtung gegenüberliegenden Seite der Aufnahmeröhre angeordnet sein. Die Barrierenströmung kann quer zu einer Längsrichtung der Aufnahmeröhre verlaufen.

Durch die Weiterbildung ergibt sich der Vorteil, dass durch die Luftbarriere, ein Luftaustausch zwischen der Luft der Aufnahmeröhre und der Umgebung durch die Aufnahmeöffnung reduziert oder unterbunden wird. Dadurch kann ein Risiko vermindert werden, dass in der Luft der Aufnahmeröhre befindliche Aerosole die Aufnahmeröhre an der Aufnahmeöffnung verlassen.

Eine Weiterbildung der Erfindung sieht vor, dass die Ausgabeeinrichtung zumindest eine Freistrahldüse umfasst. Mit anderen Worten umfasst die Ausgabeeinrichtung zur Ausgabe der dekontaminierten Luft in die Aufnahmeröhre eine oder mehrere Freistrahldüsen. Die Freistrahldüse ist dazu eingerichtet, die ausgegebene dekontaminierte Luft in einer vorgegebenen Strahlrichtung auszugeben. Es kann beispielsweise vorgesehen sein, dass die Ausgabeeinrichtung in die Aufnahmeröhre hineinragt. Die Freistrahldüse kann dazu eingerichtet sein den Strahl in die Längsrichtung der Aufnahmeröhre auszugeben. Dadurch kann die Dekontaminierte Luft beispielsweise schon bei Ausgabe in die Hauptstromrichtung ausgegeben werden.

Eine Weiterbildung der Erfindung sieht vor, dass die Luftbarriereneinrichtung dazu eingerichtet ist, den Teil der dekontaminierten Luft zur Bereitstellung der Luftbarriere in der Aufnahmeröhre an einer in der Aufnahmeröhre angeordneten Führungsschieneneinrichtung der Bildgebungsvorrichtung auszugeben. Mit anderen Worten weist die Bildgebungsvorrichtung die Führungsschieneneinrichtung auf, welche dazu eingerichtet ist, eine Auflage für ein Untersuchungsobjekt oder einen Patienten in die Aufnahmeröhre zu führen. Die Führungsschieneneinrichtung kann beispielsweise zwei Führungsschienen umfassen, die an einer Unterseite der Aufnahmeröhre oder allgemein einer unteren Hälfte der Aufnahmeröhre angeordnet sein können und entlang der Längsrichtung der Aufnahmeröhre verlaufen können. Die Luftbarriereneinrichtung ist dazu eingerichtet, die Luft zur Erzeugung der Luftbarriere ganz oder zumindest teilweise über die Führungsschieneneinrichtung auszugeben. Es kann beispielsweise vorgesehen sein, dass die Luftkanaleinrichtung dazu eingerichtet ist, die Luft der Luftbarriereneinrichtung zuzuführen, wobei die Luftbarriereneinrichtung einen Kanal aufweisen kann, der durch die Führungsschieneneinrichtung zu einer an oder in der Führungsschieneneinrichtung angeordneten Öffnung der Luftbarriereneinrichtung führt, an welcher die Luft zur Erzeugung der Luftbarriere in die Aufnahmeröhre ausgegeben wird.

Eine Weiterbildung der Erfindung sieht vor, dass die Luftkanaleinrichtung dazu eingerichtet ist, den Hauptluftstrom innerhalb einer oberen Hälfte der Aufnahmeröhre zu führen. Mit anderen Worten ist es vorgesehen, dass der Hauptluftstrom einen Verlauf aufweist, welcher oberhalb einer horizontalen Ebene verläuft, die sich in einer Mitte der Aufnahmeröhre befindet.

Eine Weiterbildung der Erfindung sieht vor, dass die Luftkanaleinrichtung dazu eingerichtet ist, den Hauptluftstrom innerhalb einer oberen Hälfte der Aufnahmeröhre zu führen. Mit anderen Worten ist es vorgesehen, dass der Hauptluftstrom einen Verlauf aufweist, welcher oberhalb einer horizontalen Ebene verläuft, die sich in einer Mitte der Aufnahmeröhre befindet. Dadurch ergibt sich der Vorteil, dass der Luftstrom über einen Patienten geführt werden kann.

Eine Weiterbildung der Erfindung sieht vor, dass die Luftkanaleinrichtung dazu eingerichtet ist, eine Ausgabe der dekontaminierten Luft an der Ausgabeeinrichtung und das Ansaugen der Luft an der Ansaugeirichtung derart einzuregeln, dass der Hauptluftstrom durch die Aufnahmeröhre eine laminare Strömungscharakteristik aufweist. Mit anderen Worten ist die Luftkanaleinrichtung dazu eingerichtet, sowohl die Ausgabe der dekontaminierten Luft an der Ausgabeeinrichtung einzustellen, als auch das Ansaugen der Luft an der Ansaugeinrichtung einzustellen. Die Luftkanaleinrichtung ist dazu eingerichtet, die Ausgabe und das Ansaugen der Luft derart einzuregeln, dass der Hauptluftstrom die laminare Strömungscharakteristik aufweist. Die laminare Strömungscharakteristik beschreibt, dass die Luft entlang dem Hauptluftstrom hauptsächlich laminar strömt. Die laminare Strömungscharakteristik betrifft das primäre Strömungsverhalten, abgesehen von unvermeidbaren Verwirbelungen an Randbereichen. Es kann beispielsweise vorgesehen sein, dass in einer Steuereinheit der Luftkanaleinrichtung Werte für Ausgabe- und Ansauggeschwindigkeiten gespeichert sind, welche erforderlich sind, damit der Hauptluftstrom die laminare Strömungscharakteristik aufweist. Die Werte können beispielsweise vorab in einer Simulation eines Strömungsverhaltens ermittelt worden sein. Durch die Weiterbildung ergibt sich der Vorteil, dass Verwirbelungen der Luft verhindert werden können.

Eine Weiterbildung der Erfindung sieht vor, dass die Luftkanaleinrichtung eine weitere Ansaugeinrichtung umfasst, die dazu eingerichtet ist, Luft aus einer Umgebung der Bildgebungsvorrichtung anzusaugen, wobei die Luftkanaleinrichtung dazu eingerichtet ist, die aus der Umgebung angesogene Luft zur Dekontamination durch die Luftfiltereinrichtung der Luftkanaleinrichtung zuzuführen. Mit anderen Worten ist die Luftkanaleinrichtung dazu eingerichtet, die Luft der Umgebung der Bildgebungsvorrichtung an der weiteren Ansaugeinrichtung anzusaugen. Durch die Weiterbildung ergibt sich der Vorteil, dass beispielsweise eine Dekontamination der Luft der Umgebung ermöglicht werden kann oder zusätzliche Luft zur Bereitstellung der Luftbarriere angesogen werden kann.

Eine Weiterbildung der Erfindung sieht vor, dass die Luftkanaleinrichtung eine weitere Ausgabeeinrichtung umfasst, die dazu eingerichtet ist, zumindest einen Teil der dekontaminierten Luft in eine Umgebung der Bildgebungsvorrichtung auszugeben. Mit anderen Worten ist die Luftkanaleinrichtung dazu eingerichtet, zumindest einen Teil der dekontaminierten Luft an der weiteren Ausgabeeinrichtung in die Umgebung der Bildgebungsvorrichtung auszugeben.

Eine Weiterbildung der Erfindung sieht vor, dass die Luftfiltereinrichtung als Plasma-Filtereinrichtung eingerichtet ist. Mit anderen Worten ist die Luftfiltereinrichtung dazu eingerichtet, ein Plasma zur Dekontamination der Durchgeleiteten Luft zu erzeugen. Das Plasma kann dabei dazu vorgesehen sein, UV-C-Strahlen zu emittieren, durch welche die Luft dekontaminiert wird.

Eine Weiterbildung der Erfindung sieht vor, dass die Luftfiltereinrichtung Filtereinrichtung, zumindest eine Elektrodeneinrichtung aufweist. Bei der Plasma-Filtereinrichtung handelt es sich um eine Filtereinrichtung zum Filtern eines Gases, beispielsweise zum Filtern von Luft, wobei zum Filtern des Gases ein Plasma erzeugt wird. Durch das erzeugte Plasma wird UV-Strahlung abgegeben, durch welche beispielsweise in dem Gas befindliche Aerosole oder Keime inaktiviert werden können, abgetötet oder Partikel deaktiviert werden können.

Es ist vorgesehen, dass die Elektrodeneinrichtung eine erste plan ausgebildete Kompositelektrode und eine zweite plan ausgebildete Kompositelektrode aufweist. Die Kompositelektroden können beispielsweise in antisymmetrischer elektromagnetischer Potentialkontur angeordnet sein. Mit anderen Worten umfasst die Elektrodeneinrichtung die erste Kompositelektrode und die zweite Kompositelektrode. Die Kompositelektroden der Elektrodeneinrichtung können insbesondere als Flächenelemente eingerichtet sein. Es ist vorgesehen, dass die Kompositelektroden der Elektrodeneinrichtung coplanar zueinander in einer Hauptflächenebene der Elektrodeneinrichtung angeordnet und durch einen Entladungsspalt räumlich voneinander getrennt sind. Mit anderen Worten befinden sich die Kompositelektroden der Elektrodeneinrichtung in der Hauptflächenebene der Elektrodeneinrichtung. Zwischen den Kompositelektroden der Elektrodeneinrichtung befindet sich ein Entladungsspalt. Bei dem Entladungsspalt handelt es sich um einen durch die Kompositelektroden gebildeten Spalt der Elektrodeneinrichtung zum Durchleiten des zu filternden Gases. Es ist vorgesehen, dass jede der Kompositelektroden ein jeweiliges Elektrodenblech aufweist, das zumindest an einer Grenzfläche des jeweiligen Elektrodenblechs zu dem Entladungsspalt eine jeweilige dielektrische Beschichtung aufweist. Mit anderen Worten sind die Elektroden als Komposite bereitgestellt, welche das jeweilige Elektrodenblech und eine auf dem jeweiligen Elektrodenblech befindliche dielektrische Beschichtung umfassen. Die dielektrische Beschichtung ist zumindest an einer von der jeweiligen Kompositelektrode an dem Entladungsspalt angrenzende Grenzfläche auf dem Elektrodenblech aufgetragen.

Die Filtereinrichtung weist eine Spannungsquelle auf, die dazu eingerichtet ist, eine Wechselspannung an der Elektrodeneinrichtung bereitzustellen wobei die Wechselspannung dazu parameterisiert ist, eine Bildung eines Plasmas durch eine dielektrische Barriereentladung in dem Entladungsspalt hervorzurufen. Mit anderen Worten ist es vorgesehen, dass die Filtereinrichtung die Spannungsquelle aufweist. Die Spannungsquelle ist dazu vorgesehen, die Wechselspannung an der Elektrodeneinrichtung bereitzustellen, um die Bildung des Plasmas in dem Entladungsspalt hervorzurufen.

Die Filtereinrichtung ist dazu eingerichtet, ein Gas entlang einer Filterströmungsrichtung, welche parallel zu einer Normalen der Hauptflächenebene der Elektrodeneinrichtung ausgerichtet ist, durch den Entladungsspalt zu führen. Mit anderen Worten weist die Filtereinrichtung strömungstechnische Führungselemente, beispielsweise Röhrenelemente auf, die dazu eingerichtet sind, eine Filterströmungsrichtung des Gases der Art zu beeinflussen oder festzulegen, dass das Gas parallel zu der Normalen der Hauptflächenebene durch den Entladungsspalt geleitet wird.

Die Filterströmungsrichtung verläuft dabei parallel zu der Normalen der Hauptflächenebene der Elektrodeneinrichtung. Mit anderen Worten verläuft die Hauptströmungsrichtung senkrecht zu der Elektrodeneinrichtung. Das Gas strömt somit senkrecht durch die Elektrodeneinrichtung durch den Entladungsspalt. Aufgrund des in dem Entladungsspalt durch die dielektrische Entladung erzeugten Plasmas, erfolgt eine Dekontamination des Gases im Bereich des Entladungsspalts. Die Dekontamination kann durch UVC-Strahlen erfolgen, die durch das Plasma abgestrahlt werden kann und/oder von Ozon, das sich im Entladungsspalt bilden kann, wenn es sich bei dem Gas um Luft handelt.

Durch die Weiterbildung ergibt sich der Vorteil, dass durch den Entladungsspalt eine Partikelfalle bereitgestellt ist, in welcher Partikel länger verbleiben als Moleküle des Gases selbst. Aufgrund des längeren Verbleibs der Partikel im Bereich des Entladungsspalts sind diese über einen längeren Zeitraum Plasmawirkungen wie Radikale und UVC ausgesetzt, wodurch eine Wahrscheinlichkeit einer Inaktivierung der Partikel zunimmt (Beispiel: bei einer UVC-Leistung von 100W/m2 und einer Verweilzeit nur von 1s ergibt sich eine Dosisleistung von 100 J/m2. Coronaviren sind ab 37 J/m2 schon zu 90% vollständig inaktiviert).

Eine Weiterbildung der Erfindung sieht vor, dass die Luftfiltereinrichtung in einer elektromagnetischen Abschirmröhre und/oder einem elektromagnetischen Abschirmbehälter angeordnet ist. Mit anderen Worten ist die Luftfiltereinrichtung zumindest teilweise durch eine Abschirmröhre und/oder einem Abschirmbehälter zum Abschirmen elektromagnetischer Strahlung und statischer Felder angeordnet. Durch die Weiterbildung ergibt sich der Vorteil, dass elektromagnetische Strahlung, welche durch die Luftfiltereinrichtung abgestrahlt werden kann, die Messungen der Bildgebungsvorrichtung nicht beeinträchtigt.

Ein zweiter Aspekt der Erfindung umfasst eine Luftkanaleinrichtung für eine Bildgebungsvorrichtung. Die Luftkanaleinrichtung ist dazu eingerichtet, Luft aus einer Aufnahmeröhre an einer Ansaugeinrichtung der Luftkanaleinrichtung anzusaugen, die angesogene Luft zur Dekontamination durch eine Luftfiltereinrichtung der Luftkanaleinrichtung zu leiten und die dekontaminierte Luft an einer Ausgabeeinrichtung der Luftkanaleinrichtung in die Aufnahmeröhre auszugeben. Die Luftkanaleinrichtung kann zur integrierten Anordnung in der Bildgebungsvorrichtung oder als Nachrüstelement für bestehende Bildgebungsvorrichtungen vorgesehen sein.

Weitere Ausführungsformen der erfindungsgemäßen Luftkanaleinrichtung folgen aus den verschiedenen Ausführungsformen der erfindungsgemäßen Bildgebungsvorrichtung.

Ein dritter Aspekt der Erfindung betrifft ein Verfahren zum Betreiben einer Bildgebungsvorrichtung, aufweisend eine Aufnahmeröhre zur Aufnahme eines durch die Bildgebungsvorrichtung zu untersuchenden Objektes. Das Verfahren umfasst ein Ansaugen von Luft aus der Aufnahmeröhre durch eine Ansaugeinrichtung der Luftkanaleinrichtung, ein Durchleiten der angesogenen Luft durch eine Luftfiltereinrichtung der Luftkanaleinrichtung zur Dekontamination der Luft durch die Luftkanaleinrichtung, und ein Ausgeben der dekontaminierten Luft an einer Ausgabeeinrichtung der Luftkanaleinrichtung.

Es ist vorgesehen, dass ein Hauptluftstrom der Luft durch die Luftkanaleinrichtung von der Ausgabeeinrichtung zu der Ansaugeinrichtung entlang der Aufnahmeröhre geführt wird, wobei der Hauptluftstrom durch die Aufnahmeröhre in eine Richtung einer Aufnahmeöffnung der Aufnahmeröhre verläuft.

Weitere Ausführungsformen des erfindungsgemäßen Verfahrens folgen aus den verschiedenen Ausführungsformen der erfindungsgemäßen Bildgebungsvorrichtung sowie der erfindungsgemäßen Luftkanaleinrichtung.

Für Anwendungsfälle oder Anwendungssituationen, die sich bei dem Verfahren ergeben können und die hier nicht explizit beschrieben sind, kann vorgesehen sein, dass gemäß dem Verfahren eine Fehlermeldung und/oder eine Aufforderung zur Eingabe einer Nutzerrückmeldung ausgegeben und/oder eine Standardeinstellung und/oder ein vorbestimmter Initialzustand eingestellt wird.

Die Erfindung wird im Folgenden anhand konkreter Ausführungsbeispiele und zugehöriger schematischer Zeichnungen näher erläutert. In den Figuren können gleiche oder funktionsgleiche Elemente mit denselben Bezugszeichen versehen sein. Die Beschreibung gleicher oder funktionsgleicher Elemente wird gegebenenfalls nicht notwendigerweise bezüglich verschiedener Figuren wiederholt. In den Figuren zeigen:
FIG 1 eine schematische Darstellung einer Bildgebungsvorrichtung;
FIG 2 eine schematische Darstellung einer Aufnahmeröhre mit einer Führungsschieneneinrichtung;
FIG 3 eine schematische Darstellung einer Strömungsgeschwindigkeit entlang der Aufnahmeröhre;
FIG 4 eine schematische Darstellung einer Strömungsgeschwindigkeit entlang der Aufnahmeröhre bei einer Bereitstellung einer Luftbarriere; und
FIG 5 eine schematische Darstellung einer Filtereinrichtung.

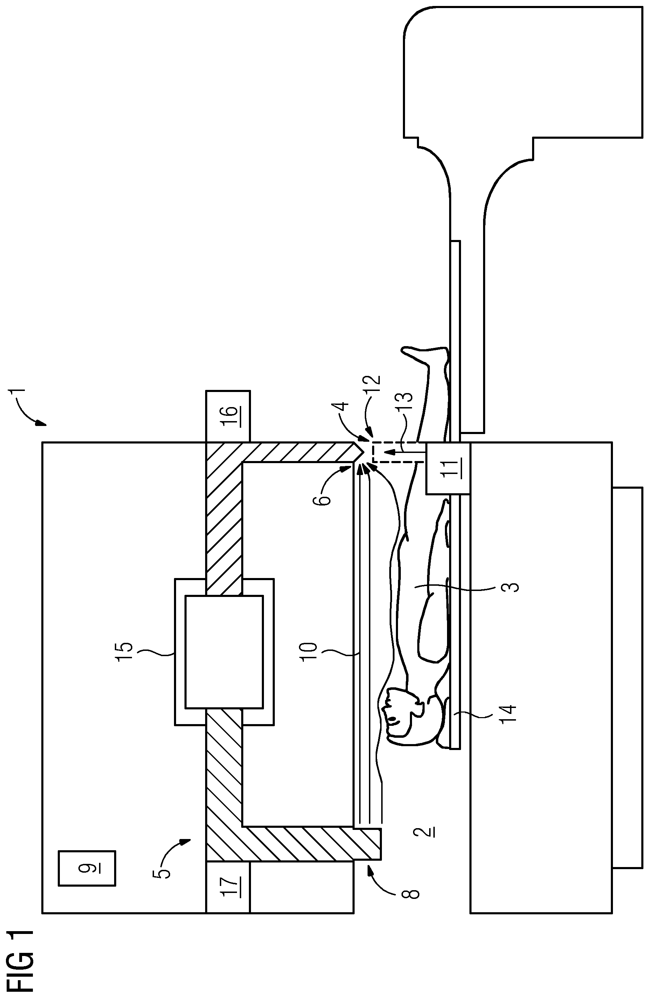

In FIG 1 ist eine schematische Darstellung einer Bildgebungsvorrichtung 1 gezeigt.

Bei der Bildgebungsvorrichtung 1 kann es sich beispielsweise um eine Bildgebungsvorrichtung 1 zur Computertomografie oder zur Magnetresonanztomographie handeln. Die Bildgebungsvorrichtung 1 kann eine Aufnahmeröhre 2 aufweisen, die zur Aufnahme eines zu untersuchenden Objekts 3 vorgesehen sein kann. Zur Untersuchung des Objektes 3 kann es erforderlich sein, das zu untersuchende Objekt 3 durch eine Aufnahmeöffnung 4 der Aufnahmeröhre 2 in die Aufnahmeröhre 2 zu verfahren. Bei dem Objekt 3 kann es sich beispielsweise um einen Patienten handeln.

Aufgrund eines Aufenthalts des Patienten in der Aufnahmeröhre 2 kann es erforderlich sein, den Patienten mit Luft zu versorgen. Zu diesem Zweck kann die Bildgebungsvorrichtung 1 eine Luftkanaleinrichtung 5 aufweisen. Dabei kann es erforderlich sein, kontaminierte Luft, welche beispielsweise durch den Patienten abgegeben werden kann, zu dekontaminieren, um eine Belastung des Patienten und/oder einen Niederschlag von Keimen auf einer Oberfläche der Aufnahmeröhre 2 zu vermeiden. Zugleich kann es erforderlich sein, ein Entweichen der kontaminierten Luft aus der Aufnahmeöffnung 4 der Aufnahmeröhre 2 in eine Umgebung der Bildgebungsvorrichtung 1 zu verhindern.

Zu diesem Zweck kann es vorgesehen sein, dass die Luftkanaleinrichtung 5 eine Ansaugeinrichtung 6 aufweist, welche dazu eingerichtet sein kann, Luft aus der Aufnahmeröhre 2 anzusaugen. Die Luft kann beispielsweise Aerosole enthalten, wodurch es erforderlich sein kann, die Luft zur Dekontamination durch eine Luftfiltereinrichtung 7 der Luftkanaleinrichtung 5 zu führen. Die Ansaugeinrichtung 6 kann bevorzugt in einer Nähe der Aufnahmeöffnung 4 der Aufnahmeröhre 2 angeordnet sein, um das Entweichen der kontaminierten Luft aus der Aufnahmeröhre 2 zu verhindern. Die Ansaugeinrichtung 6 kann insbesondere zur Anordnung an einer oberen Hälfte der Aufnahmeröhre 2 vorgesehen sein. Die Luftfiltereinrichtung 7 kann beispielsweise als Plasmafilter eingerichtet sein, welcher dazu eingerichtet ist, die kontaminierte Luft mittels einer Erzeugung von UV-Strahlung durch das Plasma 28 zu dekontaminieren. Die Luftkanaleinrichtung 5 kann dazu eingerichtet sein, die dekontaminierte Luft nach dem Verlassen der Luftfiltereinrichtung 7 einer Ausgabeeinrichtung 8 zuzuführen, um die dekontaminierte Luft in die Aufnahmeröhre 2 auszugeben.

Die Ausgabeeinrichtung 8 kann an einer der Aufnahmeöffnung 4 abgewandten Seite der Aufnahmeröhre 2 angeordnet sein, sodass die dekontaminierte Luft in einem Bereich der Aufnahmeröhre 2 ausgegeben wird, in welchem sich gewöhnlicherweise das Gesicht des Patienten befindet. Die Ansaugeinrichtung 6 und die Ausgabeeinrichtung 8 können derart angeordnet sein, dass ein Hauptluftstrom 10 der Luft von der Ausgabeeinrichtung 8 zu der Ansaugeinrichtung 6 durch die Aufnahmeröhre 2 in Richtung der Aufnahmeöffnung 4 der Aufnahmeröhre 2 verläuft. Der Hauptluftstrom 10 kann insbesondere in einer oberen Hälfte der Aufnahmeröhre 2 verlaufen. Die Luftkanaleinrichtung 5 kann eine Steuereinheit 9 aufweisen, welche die Ansaugeinrichtung 6, die Luftfiltereinrichtung 7 und die Ausgabeeinrichtung 8 ansteuern kann, um einen Umfang eines Luftstroms zu beeinflussen. Dadurch kann es beispielsweise möglich sein, die Ausgabeeinrichtung 8 und die Ansaugeinrichtung 6 derart anzusteuern, dass die entlang dem Hauptluftstrom 10 verlaufende Luft eine laminare Strömungscharakteristik aufweist. Zu diesem Zweck können Steuerwerte in der Steuereinheit 9 gespeichert sein, welche in der Ausgabeeinrichtung 8 und der Ansaugeinrichtung 6 einzuregeln sind, um die laminare Strömungscharakteristik des Hauptstroms zu ermöglichen. Die Ansaugeinrichtung 6 kann in Bezug auf eine Längsrichtung der Aufnahmeröhre 2 zwischen der Aufnahmeöffnung 4 und der Ausgabeeinrichtung 8 angeordnet sein, um ein Verlassen der Luft des Hauptstroms aus der Aufnahmeöffnung 4 zu vermeiden.

Die Luftkanaleinrichtung 5 kann eine Luftbarriereneinrichtung 11 aufweisen, welche dazu eingerichtet sein kann, eine Luftbarriere 12 zwischen der Ansaugeinrichtung 6 und der Aufnahmeöffnung 4 zu erzeugen. Dadurch kann ein Austreten der Luft aus der Aufnahmeöffnung 4 verhindert werden. Zur Bereitstellung der Luftbarriere 12 kann es vorgesehen sein, dass durch die Luftbarriereneinrichtung 11 zumindest ein Teil der dekontaminierten Luft entlang eine vorgegebene Richtung zur Erzeugung eines vorgegebenen Barrierenstroms 13 ausgegeben wird. Durch den Barrierenstrom 13 kann eine Luftzirkulation hervorgerufen werden, welche zu der Bildung der Luftbarriere 12 führen kann. Die Luftbarriereneinrichtung 11 kann dazu eingerichtet sein, den Barrierenstrom 13 über eine Führungsschieneneinrichtung 14 der Bildgebungsvorrichtung 1 auszugeben.

Es kann vorgesehen sein, dass die Luftkanaleinrichtung 5 eine weitere Ansaugeinrichtung 16 aufweist, welche dazu eingerichtet sein kann, Luft aus der Umgebung der Bildgebungsvorrichtung 1 anzusaugen. Die aus der Umgebung angesogene Luft kann ebenfalls durch die Luftkanaleinrichtung 5 zur Dekontamination durch die Luftfiltereinrichtung 7 geführt werden. Dadurch kann es vorgesehen sein, zusätzliche Luft aus der Umgebung anzusaugen, um beispielsweise den Barrierenstrom 13 bereitzustellen. Die Luftkanaleinrichtung 5 kann eine weitere Ausgabeeinrichtung 17 aufweisen, durch welche ein Teil der dekontaminierten Luft der Umgebung zugeführt werden kann.

Die Luftkanaleinrichtung 5 kann einen Abschirmbehälter 15 aufweisen, welcher die Luftfiltereinrichtung 7 zumindest teilweise umschließt, um eine Beeinträchtigung der Messung der Bildgebungsvorrichtung 1 durch elektromagnetische Strahlung, welche durch die Luftfiltereinrichtung 7 ausgegeben werden kann, zu verhindern.

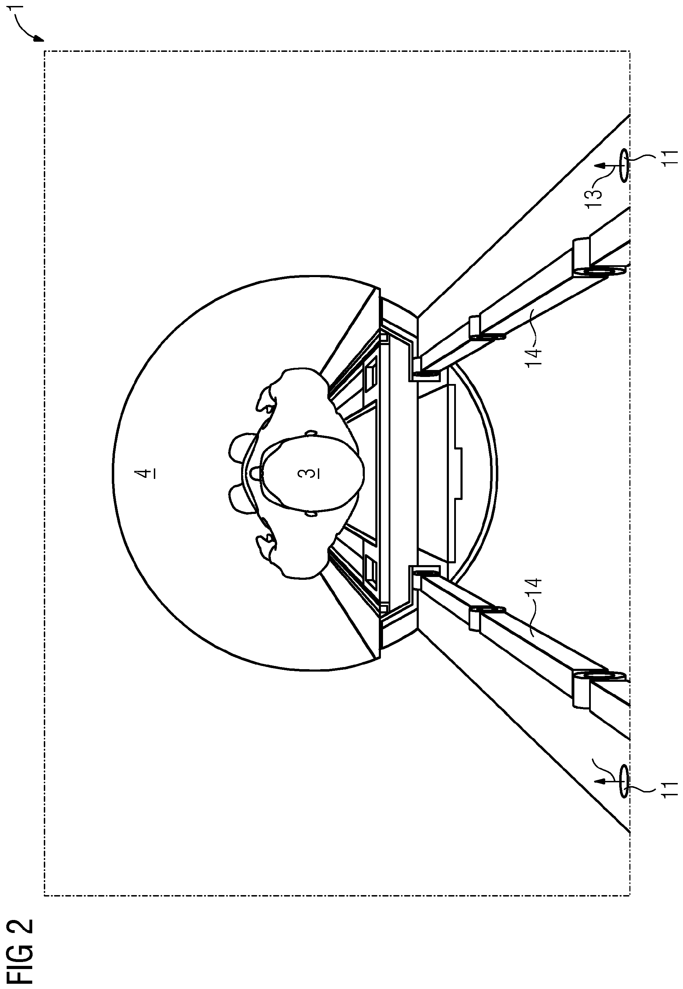

In FIG 2 ist eine schematische Darstellung einer Aufnahmeröhre 2 mit einer Führungsschieneneinrichtung 14 gezeigt.

Es kann beispielsweise vorgesehen sein, dass die Luftbarriereneinrichtung 11 zwei Öffnungen umfasst, durch welche ein Teil der dekontaminierten Luft in vorgegebene Richtungen ausgegeben werden kann, um den Barrierenstrom 13 zu bilden. Der Barrierenstrom 13 kann beispielsweise entlang einer Querrichtung der Aufnahmeröhre 2 geführt werden, um die Luftbarriere 12 entlang der Querrichtung der Aufnahmeröhre 2 zu bilden.

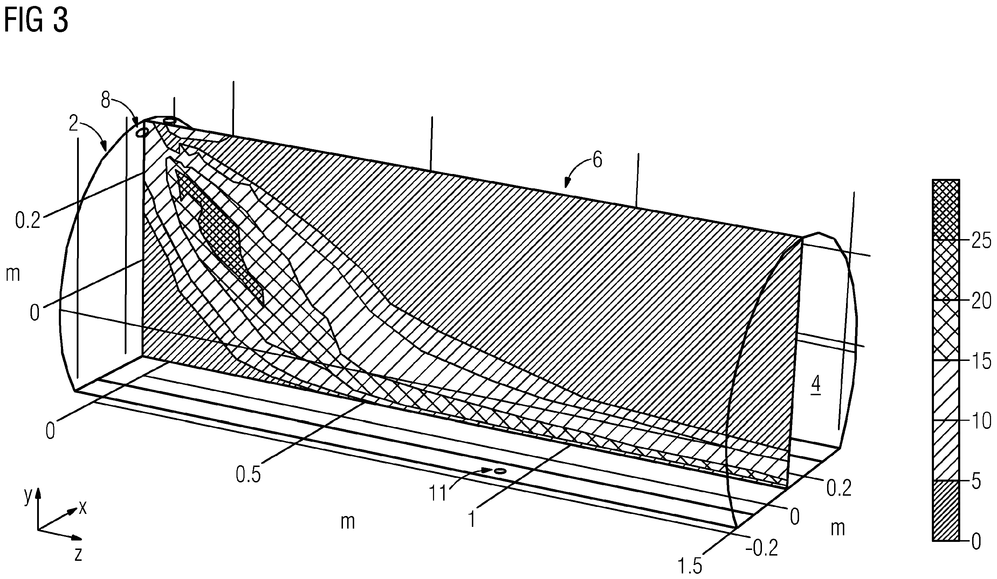

In FIG 3 ist eine schematische Darstellung einer Strömungsgeschwindigkeit entlang der Aufnahmeröhre 2 gezeigt.

Gezeigt ist ein Bereich erhöhter Geschwindigkeit der Luft ausgehend von der Ausgabeeinrichtung 8 der Luftkanaleinrichtung 5 in der Aufnahmeröhre 2.

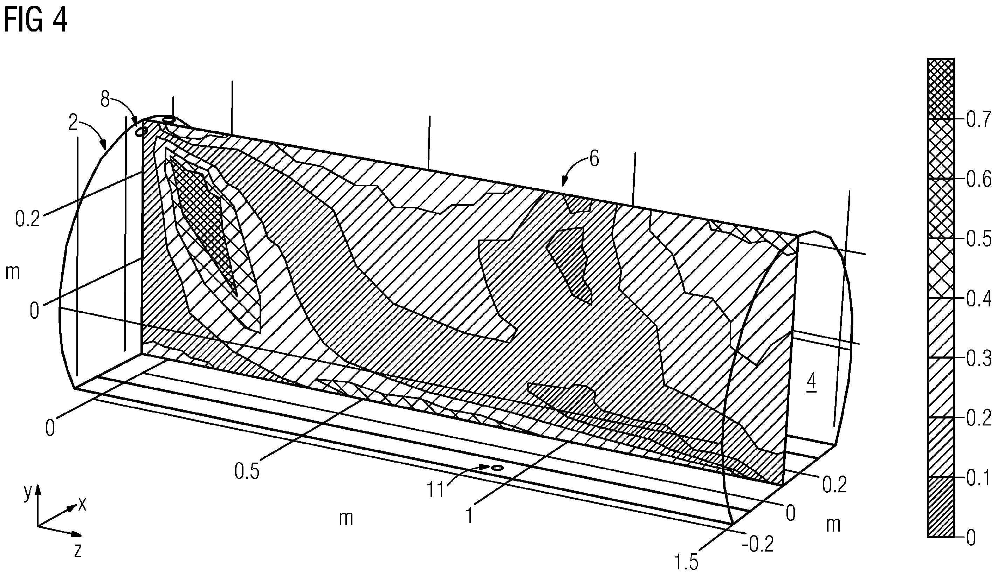

In FIG 4 ist eine schematische Darstellung einer Strömungsgeschwindigkeit entlang der Aufnahmeröhre 2 bei einer Bereitstellung einer Luftbarriere 12 gezeigt.

FIG 4 zeigt die Simulationsergebnisse für die Luftzirkulation in der Aufnahmeröhre 2 durch die Luftbarriere 12. Es ist zu erkennen, dass diese Anordnung verhindert, dass kontaminierte Luft aus der Aufnahmeröhre 2 transportiert wird.

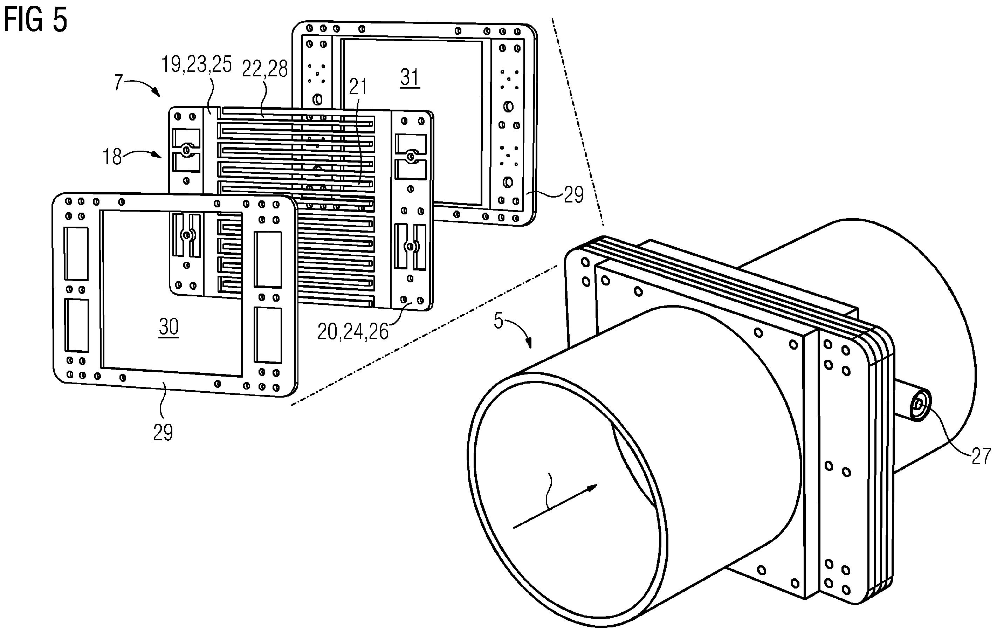

FIG 5 zeigt eine schematische Darstellung einer Luftfiltereinrichtung 7.

Die Luftfiltereinrichtung 7 kann eine Halteeinrichtung 29 aufweisen, welche dazu vorgesehen sein kann, die Elektrodeneinrichtungen 18, den Staubfilter 30 und den Aktivkohlefilter 31 in vorgegebenen Lagen anzuordnen. An der Halteinrichtung kann ein Einspeisepunkt angeordnet sein, welcher dazu vorgesehen sein kann, die Elektrodeneinrichtungen 18 mit der Wechselspannung der Spannungsquelle 27 zu versorgen. Um ein Durchleiten der Luft entlang der Filterströmungsrichtung 32 zu erreichen, können an der Luftfiltereinrichtung 7 ein oder zwei Röhren angeordnet sein, welche über einen Flansch an der Halteeinrichtung 29 angeordnet sein können.

Die Elektrodeneinrichtung 18 der Luftfiltereinrichtung 7 kann eine erste Kompositelektrode 19 und eine zweite Kompositelektrode 20 aufweisen. Die erste Kompositelektrode 19 und die zweite Kompositelektrode 20 können in einer Hauptflächenebene 21 der Elektrodeneinrichtung 18 liegen und plan sein. Die erste Kompositelektrode 19 und die zweite Kompositelektrode 20 können beispielsweise coplanar zueinander angeordnet sein.

Die Kompositelektroden 19, 20, können aus einem Ursprungsblech gefertigt worden sein, welches durch eine Bereitstellung eines Entladungsspaltes 22 in dem Ursprungsblech in ein erstes Elektrodenblech 23 und ein zweites Elektrodenblech 24 aufgeteilt worden sein kann, wobei das erste Elektrodenblech 23, 24 und das zweite Elektrodenblech 23, 24 durch den Entladungsspalt 22 voneinander getrennt sein können. Die beiden Kompositelektroden 19, 20, können ein jeweiliges Elektrodenblech 23, 24 aufweisen, welches mit einer jeweiligen dielektrischen Beschichtung 25, 26, beschichtet sein kann, wodurch es sich bei den jeweiligen Elektroden um Komposite handeln kann. Das Elektrodenblech 23, 24 der jeweiligen Kompositelektrode 19, 20 kann beispielsweise Aluminium, eine Aluminiumlegierung und/oder rostfreien Stahl als Material aufweisen. Die dielektrische Beschichtung 25, 26, der jeweiligen Kompositelektrode 19, 20 kann zumindest an einer Grenzfläche der jeweiligen Kompositelektrode 19, 20 zu dem Entladungsspalt 22 auf dem jeweiligen Elektrodenblech 23, 24 aufgetragen sein. Die Aufgabe der dielektrischen Beschichtung 25, 26, kann darin bestehen, eine dielektrische Entladung in dem Entladungsspalt 22 zu ermöglichen, wenn eine entsprechend parameterisierte elektrische Wechselspannung an der Elektrodeneinrichtung 18 angelegt ist. Die dielektrische Beschichtung 25, 26, kann einen oder mehrere Polymere als Material aufweisen. Die möglichen Polymere können beispielsweise Fluorkunststoffe, insbesondere Polyvinylidendifluorid und/oder Polytetrafluorethylen aufweisen. Dem zumindest einen Polymer kann auch Graphitfluorid beigemischt sein. Die dielektrische Beschichtung 25, 26, kann auch eine oder mehrere Keramiken als Material, insbesondere Bariumtitanat aufweisen.

In der folgenden Beschreibung wird der Begriff Luftfiltereinrichtung 7 für einen Aufbau verwendet, der eine Filtereinheit, eine Stromversorgung, eine Abschirmung zur Abschirmung elektromagnetischer Strahlen und alle relevanten Kabel und sonstige Anschlüsse umfasst. Die Luftfiltereinrichtung 7 kann auch als Dekontaminationseinrichtung bezeichnet werden.

Die Luftfiltereinrichtung 7 kann zur Anordnung in einer Luftkanaleinrichtung 5 zur Reinigung von Luft/Aerosolen in der Aufnahmeröhre 2 der Bildgebungsvorrichtung 1 vorgesehen sein.

Es kann vorgesehen sein, ein vorgegebenes Strömungsmuster in der Aufnahmeröhre 2 der Bildgebungsvorrichtung 1 zu erzeugen, um zu verhindern, dass kontaminierte Luft aus der Aufnahmeröhre 2 nach außen gelangt. Das Strömungsmuster kann eine Luftbarriere 12 umfassen, welche auch als Luftschleier bekannt ist.

Die Luftfiltereinrichtung 7 kann eine Dekontamination von Luft mittels Plasma 28 ermöglichen. Die Luftfiltereinrichtung 7 kann mit einer relativ hohen Wirksamkeit und maßgeschneiderter Geometrie in die bestehende Luftkanaleinrichtung 5 einer Bildgebungsvorrichtung 1 angeordnet sein. Alternativ dazu kann es möglich sein, dass die Luftfiltereinrichtung 7 als separate, an die Bildgebungsvorrichtung 1 angepasste Einheit angeordnet sein kann.

Aufgrund des geringen Bauraums der Luftfiltereinrichtung 7 können effektive Maßnahmen zur Sicherstellung einer elektromagnetischen Verträglichkeit realisiert werden.

Um die Luft im Inneren der Aufnahmeröhre 2 zu dekontaminieren, kann die Ausgabeeinrichtung 8 die Ansaugeinrichtung 6 gegenüber bekannten Bildgebungsvorrichtungen 1 angepasst werden. Aufgrund von Sicherheitsaspekten, wie dem Schutz des Patienten oder des Bedieners vor Helium bei Leckage oder Quenching kann eine weitere Ansaugeinrichtung 16 6 am Boden der Bildgebungsvorrichtungen 1 angeordnet sein. Die von der Umgebung durch die weitere Ansaugeinrichtung 16 angesogene Luft kann zu einer Ausgabeeinrichtung 8 in der Aufnahmeröhre 2 direkt über den Patienten geführt werden.

Die Luftfiltereinrichtung 7 kann an jeder geeigneten Stelle der Luftkanaleinrichtung 5 platziert werden. Die Standardabmessungen der im der Luftkanaleinrichtung 5 verwendeten Rohre können beispielsweise einen Durchmesser von 55 mm aufweisen. Die Luftdurchsätze können beispielsweise bei 250 bis 300 m³/h liegen. Simulationsergebnisse, die auf diesen Anforderungen basieren, zeigen, dass eine Mindestgröße einer Elektrode der Luftfiltereinrichtung 7 von 84 x 56 x 1 mm geeignet sein könnte. Aufgrund dieser sehr geringen Baugröße lassen sich wirksame Maßnahmen zur elektromagnetischen Verträglichkeit, zur Sicherstellung der HF- und B-Feld-Verträglichkeit für die Verwendung der Luftfiltereinrichtung 7 in einer Bildgebungsvorrichtungen 1 realisieren. Dies kann ermöglicht werden, indem die Luftfiltereinrichtung 7 und ein Plasmastromgenerator der Luftfiltereinrichtung 7 in ein Metallrohr eingebaut sein kann. Das Metallrohr kann beispielsweise Edelstahl aufweisen.

Um sicherzustellen, dass kontaminierte Luft durch die Luftfiltereinrichtung 7 geleitet wird, kann eine Ansaugeinrichtung 6 an der Aufnahmeöffnung 4 der Aufnahmeröhre 2 angebracht sein. Diese Ansaugeinrichtung 6 kann hinsichtlich des Luftvolumenstroms innerhalb der Aufnahmeröhre 2 und des laminaren Luftstroms über dem Patienten so eingestellt sein, dass die kontaminierte Luft innerhalb der Aufnahmeröhre 2 vollständig ersetzt wird.

Um einen konstanten laminaren Luftstrom über dem Patienten in der Aufnahmeröhre 2 zu erzeugen, kann die Ausgabeeinrichtung 8 maßgeschneiderte Freistrahldüsen aufweisen, die auf einer in einer Längsrichtung gegenüberliegenden Seite der Ansaugeinrichtung 6 platziert sein können.

Eine räumliche Abtrennung des Luftstroms in der Aufnahmeröhre 2 kann dadurch erreicht werden, dass der sich weiter in die Richtung der Aufnahmeöffnung 4 bewegende Hauptstrom gestoppt wird. Zu diesem Zweck kann zumindest ein Teil der dekontaminierten Luft durch die Luftbarriereneinrichtung 11 in die Aufnahmeröhre 2 geleitet werden. Die Luftbarriereneinrichtung 11 kann zwei Lufteinlässe umfassen, die sich an den linken und rechten Führungsleisten der Führungsschieneneinrichtung 14 innerhalb der Aufnahmeröhre 2 befinden.

Durch die Verwendung der beschriebenen Luftfiltereinrichtung 7 kann eine Verlängerung des Wartungsintervalls des Filters gegenüber Luftfiltereinrichtungen 7 nach dem Stand der Technik, welche einen jährlichen Wechsel erfordern, ermöglicht werden.

Eine auf dem Prinzip der dielektrischen Entladung basierende Luftfiltereinrichtung 7 ist eine kostengünstige, praktisch wartungsfreie, flache, skalierbare und universell einsetzbare, energieeffiziente Plasmafiltereinheit zur Desinfektion von Luft. Diese präventive Technologie ist für den universellen Einsatz gegen luftübertragene Infektionen geeignet. Damit ist die Plasmatechnologie eine ideale, energieeffiziente Verbesserung gegenüber herkömmlichen mechanischen Filtern, wie HEPA-Filtern, welche den höchsten Differenzdruckbedarf aufweisen und der universell gegen alle Erreger wirksamen Inaktivierung von Erregern durch UV-C-Strahlung, welche große Wechselwirkungswege erfordern. Während plasmainduzierte Radikalreaktionen und UV-C-Strahlung gasförmige Verbindungen, Gerüche, Mikroorganismen und Viren inaktivieren, übernehmen konventionelle, weitgehend wartungsfreie selbststerilisierende Filterelemente die mechanische Abscheidung von Staubpartikeln und Aerosolen in einer technischen Kombination aus sich ergänzenden Einzelelementen.

Zentrale mechanische HEPA-Filter können durch die kompakte Luftfiltereinrichtung 7, welche Plasmaelektroden umfassen kann, ersetzt werden. Dadurch werden drastische Mengen an Sondermüll vermieden. Es verbleiben lediglich ein selbststerilisierender Staub- und Feuchtigkeitsvorfilter am Eingang sowie ein dünner Aktivkohlefilter 31 am Ausgang der Plasmaelektroden der Luftfiltereinrichtung 7. Zusätzliche UV-Lampen und die dazugehörige Technik können dank der Luftfiltereinrichtung 7 bei stärkerer Desinfektionswirkung eingespart werden.

## Patentansprüche

1. Bildgebungsvorrichtung (1), aufweisend eine Aufnahmeröhre (2) zur Aufnahme eines durch die Bildgebungsvorrichtung (1) zu untersuchenden Objektes (3), wobei
die Bildgebungsvorrichtung (1) eine Luftkanaleinrichtung (5) aufweist, die dazu eingerichtet ist, Luft der Aufnahmeröhre (2) an einer Ansaugeinrichtung (6) der Luftkanaleinrichtung (5) anzusaugen, die angesogene Luft zur Dekontamination durch eine Luftfiltereinrichtung (7) der Luftkanaleinrichtung (5) zu leiten und die dekontaminierte Luft an einer Ausgabeeinrichtung (8) der Luftkanaleinrichtung (5) in die Aufnahmeröhre (2) auszugeben,
**dadurch gekennzeichnet, dass**
die Luftkanaleinrichtung (5) dazu eingerichtet ist, einen Hauptluftstrom (10) der Luft von der Ausgabeeinrichtung (8) zu der Ansaugeinrichtung (6) entlang der Aufnahmeröhre (2) zu führen, wobei der Hauptluftstrom (10) durch die Aufnahmeröhre (2) in eine Richtung einer Aufnahmeöffnung (4) der Aufnahmeröhre (2) verläuft.

2. Bildgebungsvorrichtung (1) nach Anspruch 1, wobei die Ausgabeeinrichtung (8) zumindest eine Freistrahldüse umfasst.

3. Bildgebungsvorrichtung (1) nach Anspruch 1 oder 2, wobei die Luftkanaleinrichtung (5) eine Luftbarriereneinrichtung (11) aufweist, die dazu eingerichtet ist, zumindest einen Teil der dekontaminierten Luft zur Erzeugung einer Luftbarriere (12) in der Aufnahmeröhre (2) zwischen der Ansaugeinrichtung (6) und der Aufnahmeöffnung (4) auszugeben.

4. Bildgebungsvorrichtung (1) nach Anspruch 3, wobei die Luftbarriereneinrichtung (11) dazu eingerichtet ist, den Teil der dekontaminierten Luft zur Bereitstellung der Luftbarriere (12) in der Aufnahmeröhre (2) an einer in der Aufnahmeröhre (2) angeordneten Führungsschieneneinrichtung (14) der Bildgebungsvorrichtung (1) auszugeben.

5. Bildgebungsvorrichtung (1) nach einem der vorhergehenden Ansprüche, wobei die
Luftkanaleinrichtung (5) dazu eingerichtet ist, den Hauptluftstrom (10) innerhalb einer oberen Hälfte der Ausnahmeröhre zu führen.

6. Bildgebungsvorrichtung (1) nach einem der vorhergehenden Ansprüche, wobei die
Luftkanaleinrichtung (5) dazu eingerichtet ist, eine Ausgabe der dekontaminierten Luft an der Ausgabeeinrichtung (8) und das Ansaugen der Luft an der Ansaugeirichtung derart einzuregeln, dass der Hauptluftstrom (10) durch die Aufnahmeröhre (2) eine laminare Strömungscharakteristik aufweist.

7. Bildgebungsvorrichtung (1) nach einem der vorhergehenden Ansprüche, wobei die Luftkanaleinrichtung (5) eine weitere Ansaugeinrichtung (16) umfasst, die dazu eingerichtet ist, Luft aus einer Umgebung der Bildgebungsvorrichtung (1) anzusaugen, wobei die Luftkanaleinrichtung (5) dazu eingerichtet ist, die aus der Umgebung angesogene Luft zur Dekontamination durch eine Luftfiltereinrichtung (7) der Luftkanaleinrichtung (5) zuzuführen.

8. Bildgebungsvorrichtung (1) nach einem der vorhergehenden Ansprüche, wobei die Luftkanaleinrichtung (5) eine weitere Ausgabeeinrichtung (17) umfasst, die dazu eingerichtet ist, zumindest einen Teil der dekontaminierten Luft in eine Umgebung der Bildgebungsvorrichtung (1) auszugeben.

9. Bildgebungsvorrichtung (1) nach einem der vorhergehenden Ansprüche, wobei die Luftfiltereinrichtung (7) als Plasmafilter eingerichtet ist.

10. Bildgebungsvorrichtung (1) nach Anspruch 9, wobei die Luftfiltereinrichtung (7) aufweist:
- zumindest eine Elektrodeneinrichtung (18), wobei die Elektrodeneinrichtung (18) eine erste plan ausgebildete Kompositelektrode (19) und eine zweite plan ausgebildete Kompositelektrode (20) aufweist, wobei
- die Kompositelektroden (19, 20) der Elektrodeneinrichtung (18) coplanar zueinander in einer Hauptflächenebene (21) der Elektrodeneinrichtung (18) angeordnet und durch einen Entladungsspalt (22) räumlich voneinander getrennt sind, und wobei
- jede der Kompositelektroden (19, 20) ein jeweiliges Elektrodenblech (23, 24) aufweist, das zumindest an einer Grenzfläche des jeweiligen Elektrodenblechs (23, 24) zu dem Entladungsspalt (22) eine jeweilige dielektrische Beschichtung (25, 26) aufweist,
- die Plasma (28)-Filtereinrichtung eine Spannungsquelle (27) aufweist, die dazu eingerichtet ist, eine Wechselspannung an der Elektrodeneinrichtung (18) bereitzustellen, wobei
- die Wechselspannung dazu parameterisiert ist, eine Bildung eines Plasmas (28) durch eine dielektrische Barriereentladung in dem Entladungsspalt (22) hervorzurufen, und
- die Plasma (28)-Filtereinrichtung dazu eingerichtet ist, die Luft entlang einer Filterströmungsrichtung (32), welche parallel zu einer Normalen der Hauptflächenebene (21) der Elektrodeneinrichtung (18) ausgerichtet ist, durch den Entladungsspalt (22) zu führen.

11. Bildgebungsvorrichtung (1) nach einem der vorhergehenden Ansprüche, wobei die Luftfiltereinrichtung (7) in einer Abschirmröhre und/oder einem Abschirmbehälter (15) angeordnet ist.

12. Verfahren zum Betreiben einer Bildgebungsvorrichtung (1), aufweisend eine Aufnahmeröhre (2) zur Aufnahme eines durch die Bildgebungsvorrichtung (1) zu untersuchenden Objektes (3),
umfassend die Schritte:
Ansaugen von Luft der Aufnahmeröhre (2) durch eine Ansaugeinrichtung (6) einer Luftkanaleinrichtung (5) der Bildgebungsvorrichtung (1),
leiten der angesogenen Luft zur Dekontamination durch eine Luftfiltereinrichtung (7) der Luftkanaleinrichtung (5) durch die Luftkanaleinrichtung (5);
Ausgeben der dekontaminierten Luft durch eine Ausgabeeinrichtung (8) der Luftkanaleinrichtung (5) in die Aufnahmeröhre (2),
**dadurch gekennzeichnet, dass**
ein Hauptluftstrom (10) der Luft durch die Luftkanaleinrichtung (5) von der Ausgabeeinrichtung (8) zu der Ansaugeinrichtung (6) entlang der Aufnahmeröhre (2) geführt wird, wobei der Hauptluftstrom (10) durch die Aufnahmeröhre (2) in eine Richtung einer Aufnahmeöffnung (4) der Aufnahmeröhre (2) verläuft.

## Claims

1. Imaging apparatus (1) having a tubular receptacle (2) for receiving an object (3) that is to be examined by the imaging apparatus (1), wherein
the imaging apparatus (1) has an air channel device (5) which is configured to draw air from the tubular receptacle (2) at an intake device (6) of the air channel device (5), to guide the drawn-in air through an air filter device (7) of the air channel device (5) for the purpose of decontamination, and to output the decontaminated air into the tubular receptacle (2) at an output device (8) of the air channel device (5),
**characterised in that**
the air channel device (5) is configured to guide a main airstream (10) of the air along the tubular receptacle (2) from the output device (8) to the intake device (6), wherein the main airstream (10) runs through the tubular receptacle (2) towards a receptacle opening (4) of the tubular receptacle (2).

2. Imaging apparatus (1) according to claim 1, wherein the output device (8) has at least one free-jet nozzle.

3. Imaging apparatus (1) according to claim 1 or 2, wherein the air channel device (5) has an air barrier device (11) which is configured to output at least a portion of the decontaminated air for the purpose of generating an air barrier (12) in the tubular receptacle (2) between the intake device (6) and the receptacle opening (4).

4. Imaging apparatus (1) according to claim 3, wherein the air barrier device (11) is configured to output the portion of the decontaminated air for providing the air barrier (12) in the tubular receptacle (2) at a guide rail device (14) of the imaging apparatus (1), said guide rail device (14) being arranged in the tubular receptacle (2).

5. Imaging apparatus (1) according to one of the preceding claims, wherein
the air channel device (5) is configured to guide the main airstream (10) within an upper half of the tubular receptacle.

6. Imaging apparatus (1) according to one of the preceding claims, wherein
the air channel device (5) is configured to adjust an output of the decontaminated air at the output device (8) and the intake of the air at the intake device in such a way that the main airstream (10) through the tubular receptacle (2) has a laminar flow characteristic.

7. Imaging apparatus (1) according to one of the preceding claims, wherein the air channel device (5) comprises a further intake device (16), which is configured to draw air from an environment of the imaging apparatus (1), wherein the air channel device (5) is configured to supply the drawn-in air from the environment for decontamination by an air filter device (7) of the air channel device (5).

8. Imaging apparatus (1) according to one of the preceding claims, wherein the air channel device (5) comprises a further output device (17), which is configured to output at least a portion of the decontaminated air into an environment of the imaging apparatus (1).

9. Imaging apparatus (1) according to one of the preceding claims, wherein the air filter device (7) is configured as a plasma filter.

10. Imaging apparatus (1) according to claim 9, wherein the air filter device (7) comprises:
- at least one electrode device (18), wherein the electrode device (18) has a first planar composite electrode (19) and a second planar composite electrode (20), wherein
- the composite electrodes (19, 20) of the electrode device (18) are arranged in a reciprocally coplanar manner in a principal surface plane (21) of the electrode device (18) and are physically separated from each other by a discharge gap (22), and wherein
- each of the composite electrodes (19, 20) has a respective electrode plate (23, 24) with a respective dielectric coating (25, 26) at least at a boundary surface of the respective electrode plate (23, 24) relative to the discharge gap (22),
- the plasma (28) filter device has a voltage source (27) which is configured to supply an alternating voltage to the electrode device (18), wherein
- the alternating voltage is parameterised to cause the formation of a plasma (28) by means of a dielectric barrier discharge in the discharge gap (22), and
- the plasma (28) filter device is configured to guide the air along a filter flow direction (32), this being oriented parallel to a normal of the principal surface plane (21) of the electrode device (18), and through the discharge gap (22).

11. Imaging apparatus (1) according to one of the preceding claims, wherein the air filter device (7) is arranged in a screening tube and/or a screening container (15).

12. Method for operating an imaging apparatus (1) which has a tubular receptacle (2) for receiving an object (3) that is to be examined by the imaging apparatus (1),
comprising the steps:
drawing air from the tubular receptacle (2) by means of an intake device (6) of an air channel device (5) of the imaging apparatus (1),
guiding the drawn-in air through the air channel device (5) for the purpose of decontamination by an air filter device (7) of the air channel device (5);
outputting the decontaminated air into the tubular receptacle (2) by means of an output device (8) of the air channel device (5),
**characterised in that**
a main airstream (10) of the air is guided along the tubular receptacle (2) from the output device (8) to the intake device (6) by means of the air channel device (5), wherein the main airstream (10) runs through the tubular receptacle (2) towards a receptacle opening (4) of the tubular receptacle (2).

## Revendications

1. Installation (1) d'imagerie, comportant un tube (2) d'enregistrement pour l'enregistrement d'un objet (3) à examiner par l'installation (1) d'imagerie, dans laquelle
l'installation (1) d'imagerie a un dispositif (5) à conduit d'air, qui est agencé pour aspirer de l'air du tube (2) d'enregistrement à un dispositif (6) d'aspiration du dispositif (5) à conduit d'air, conduire l'air aspiré pour la décontamination dans un dispositif (7) de filtration d'air du dispositif (5) à conduit d'air et envoyer l'air contaminé à un dispositif (8) de sortie du dispositif (5) à conduit d'air au tube (2) d'enregistrement,
**caractérisée en ce que**
le dispositif (5) à conduit d'air est agencé pour conduire un courant (10) principal d'air du dispositif (8) de sortie au dispositif (6) d'aspiration le long du tube (2) d'enregistrement, dans lequel le courant (10) principal d'air s'étend dans le tube (2) d'enregistrement dans une direction d'une ouverture (4) d'enregistrement du tube (2) d'enregistrement.

2. Installation (1) d'imagerie suivant la revendication 1, dans laquelle le dispositif (8) de sortie comprend au moins une buse à jet libre.

3. Installation (1) d'imagerie suivant la revendication 1 ou 2, dans laquelle
le dispositif (5) à conduit d'air a un dispositif (11) formant barrière d'air, qui est agencé pour donner au moins une partie de l'air contaminé pour la production d'une barrière (12) d'air dans le tube (2) d'enregistrement entre le dispositif (6) d'aspiration et l'ouverture (4) d'enregistrement.

4. Installation (1) d'imagerie suivant la revendication 3, dans laquelle le dispositif (11) de barrière d'air est agencé pour donner la partie de l'air contaminé, pour disposer de la barrière (12) d'air dans le tube (2) d'enregistrement, à un dispositif (14) à rail de guidage, disposé dans le tube (2) d'enregistrement de l'installation (1) d'imagerie.

5. Installation (1) d'imagerie suivant l'une des revendications précédentes, dans laquelle
le dispositif (5) à conduit d'air est agencé pour conduire le courant (10) principal d'air dans une moitié supérieure du tube d'enregistrement.

6. Installation (1) d'imagerie suivant l'une des revendications précédentes, dans laquelle
le dispositif (5) à conduit d'air est agencé pour régler une sortie de l'air décontaminé sur le dispositif (8) de sortie et l'aspiration de l'air sur le dispositif d'aspiration, de manière à ce que le courant (10) principal d'air dans le tube (2) d'enregistrement ait une caractéristique d'écoulement laminaire.

7. Installation (1) d'imagerie suivant l'une des revendications précédentes, dans laquelle le dispositif (5) à conduit d'air comprend un autre dispositif (16) d'aspiration, qui est agencé pour aspirer de l'air de l'atmosphère ambiante de l'installation (1) d'imagerie, dans laquelle le dispositif (5) à conduit d'air est agencé pour envoyer de l'air aspiré de l'atmosphère ambiante pour la décontamination dans un dispositif (7) de filtration de l'air du dispositif (5) à conduit d'air.

8. Installation (1) d'imagerie suivant l'une des revendications précédentes, dans laquelle le dispositif (5) à conduit d'air comprend un autre dispositif (17) de sortie, qui est agencé pour envoyer au moins une partie de l'air décontaminé dans une atmosphère ambiante de l'installation (1) d'imagerie.

9. Installation (1) d'imagerie suivant l'une des revendications précédentes, dans laquelle le dispositif (7) de filtration d'air est agencé sous la forme d'un filtre à plasma.

10. Installation (1) d'imagerie suivant la revendication 9, dans laquelle le dispositif (7) de filtration d'air comporte :
- au moins un dispositif (18) à électrodes, dans laquelle le dispositif (18) à électrodes a une première électrode (19) composite de constitution plane et une deuxième électrode (20) composite de constitution plane, dans laquelle
- les électrodes (19, 20) composites du dispositif (18) à électrodes sont disposées de manière coplanaire l'une par rapport à l'autre dans un plan (21) de surface principale du dispositif (18) à électrodes et sont séparées l'une de l'autre dans l'espace par un intervalle (22) de décharge, et dans laquelle
- chacune des électrodes (19, 20) composites a une tôle (23, 24) respective d'électrode, qui a au moins un revêtement (25, 26) diélectrique respectif à une surface limite de la tôle (23, 24) d'électrode respective avec l'intervalle (22) de décharge,
- le dispositif de filtration à plasma (28) a une source (27) de tension, qui est agencée pour donner une tension alternative au dispositif (18) à électrodes, dans laquelle
- la tension alternative est paramétrée pour provoquer la formation d'un plasma (28) par une décharge barrière diélectrique dans l'intervalle (22) de décharge, et
- le dispositif de filtration à plasma (28) est agencé pour conduire l'air dans l'intervalle (22) de décharge suivant une direction (32) d'écoulement du filtre, qui est parallèle à une normale au plan (21) de surface principale du dispositif (18) à électrodes.

11. Installation (1) d'imagerie suivant l'une des revendications précédentes, dans laquelle le dispositif (7) de filtration de l'air est disposé dans un tube de blindage et/ou un récipient (15) de blindage.

12. Procédé pour faire fonctionner une installation (1) d'imagerie, comportant un tube (2) d'enregistrement pour l'enregistrement d'un objet (3) à examiner par l'installation (1) d'imagerie,
comprenant les stades :
aspiration d'air du tube (2) d'enregistrement dans un dispositif (6) d'aspiration d'un dispositif (5) à conduit pour de l'air de l'installation (1) d'imagerie,
envoi de l'air aspiré dans le dispositif (5) à conduit d'air pour la décontamination par un dispositif (7) de filtration de l'air d'un dispositif (5) à conduit pour de l'air,
envoi dans le tube (2) d'enregistrement de l'air décontaminé par un dispositif (8) d'envoi du dispositif (5) à conduit pour de l'air,
**caractérisé en ce que**
l'on fait passer un courant (10) principal de l'air dans le dispositif (5) à conduit pour de l'air du dispositif (8) de sortie au dispositif (6) d'aspiration le long du tube (2) d'enregistrement, dans lequel le courant (10) principal d'air dans le tube (2) d'enregistrement s'étend dans une direction d'une ouverture (4) d'enregistrement du tube (2) d'enregistrement.
